# FASCICULE DE BREVET EUROPEEN

(11) **EP 2 785 344 B1**
(45) Date de publication et mention de la délivrance du brevet: **02.11.2016**
(21) Numéro de dépôt: 12806593.5
(22) Date de dépôt: 29.11.2012
(51) Int. Cl.: A61K 31/4458, A61P 25/00

(54) **PHACETOPERANE POUR TRAITER UN TROUBLE DEFICIT DE L'ATTENTION HYPERACTIVITE**
PHACETOPERAN ZUR BEHANDLUNG VON AUFMERKSAMKEITSDEFIZITSTÖRUNG MIT HYPERAKTIVITÄT
PHACETOPERANE FOR THE TREATMENT OF ATTENTION-DEFICIT HYPERACTIVITY DISORDER

(30) Priorité: 29.11.2011 FR 1160902
(43) Date de publication de la demande: 08.10.2014
(73) Titulaire: NLS Pharma AG, 6370 Stans (CH)
(72) Inventeur: KONOFAL, Eric, F-60300 Senlis (FR); FIGADERE, Bruno, F-91530 Saint Cheron (FR)
(74) Mandataire: Chajmowicz, Marion
(86) Numéro de dépôt international: PCT/FR2012/052749
(87) Numéro de publication internationale: WO 2013/079873

(56) Documents cités:
- US-A- 2 928 835
- DATABASE EMBASE [Online] ELSEVIER SCIENCE PUBLISHERS, AMSTERDAM, NL; 1963, LEITCH A ET AL: "A trial of pour anti-depressant drugs", XP002676742, Database accession no. EMB-0007593501 & LEITCH A ET AL: "A trial of pour anti-depressant drugs", PSYCHOPHARMACOLXXIIA 1963, vol. 4, no. 1, 1963, pages 72-77,

## Description

L'invention concerne le traitement du Trouble Déficit de l'Attention Hyperactivité.

### Arrière-plan technologique de l'invention :

Le Trouble Déficit de l'Attention Hyperactivité (TDAH) associe à une hyperactivité comportementale, une inattention, et une impulsivité. Le diagnostic repose sur des critères cliniques définis dans le Manuel Diagnostique et Statistique des Troubles Mentaux (DSM-IV TR). Le TDAH est avant tout l'expression exagérée, permanente et continue de manifestations comportementales qui ne sont pas dues à une carence éducative, pédagogique ou socio-économique.

Ce trouble est un motif fréquent de consultation en psychopathologie de l'enfant. Sa prévalence dans la population générale des enfants est selon les études de 2 à 5%. Les signes d'inattention peuvent persister au-delà de l'enfance et sont responsables de difficultés sociales, relationnelles et affectives. Jusqu'à 60% des enfants souffrant de TDAH continuent à présenter des symptômes caractéristiques à l'âge adulte, bien que la présentation typique corresponde à des critères totalement différents (travail, relations, parentalité,...). Les études ont montré que le trouble est cliniquement significatif à l'âge adulte, avec des dysfonctionnements au plan professionnel, familial et affectif. En outre, la comorbidité est élevée, et peut compliquer le diagnostic et le traitement.

La ritaline® (chlorhydrate de méthylphénidate) est actuellement le médicament le plus prescrit pour traiter le TDAH, mais il n'est pas exempt d'effets secondaires, qui peuvent être sévères.

Il existe toujours un besoin en un médicament efficace pour traiter un TDAH, qui soit de préférence moins toxique que la ritaline.

### Résumé de l'invention :

L'invention vise l'alpha-phényl(pipéridin-2-yl)méthanol, ou un de ses sels ou esters pharmaceutiquement acceptables, pour une utilisation dans le traitement d'un TDAH.

De préférence on utilise l'acétate de l'alpha-phényl(pipéridin-2-yl)méthanol sous forme thréo (phacétopérane) ou un de ses sels pharmaceutiquement acceptables.

Dans un mode de réalisation particulier, ledit phacétopérane est sous sa forme dextrogyre.

Dans un autre mode de réalisation particulier, ledit phacétopérane est sous sa forme levogyre.

Légende des Figures :

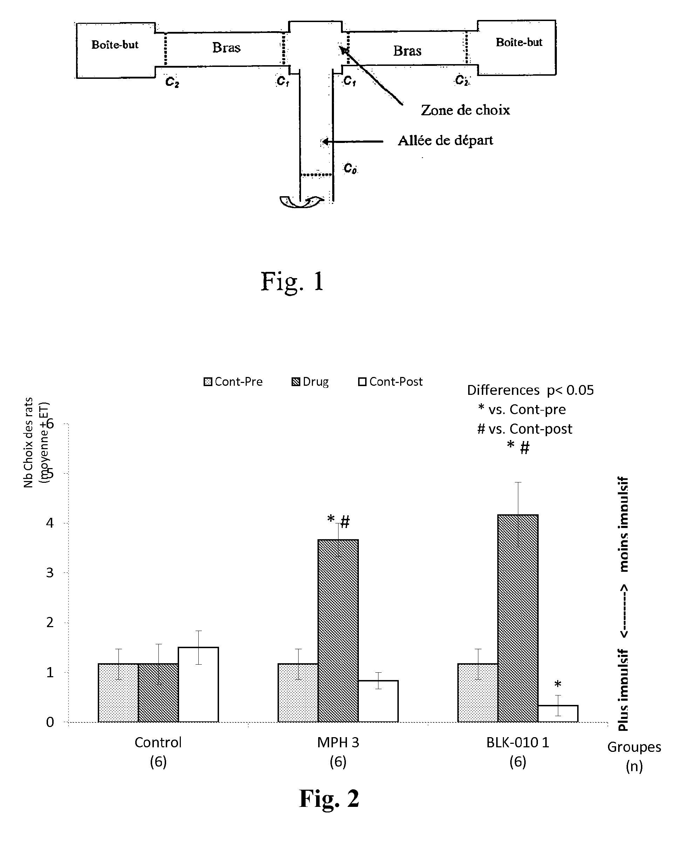

La Figure 1 est un schéma du test du labyrinthe en T : c0, c1 et c2 sont les limites des portes guillotine.
La Figure 2 présente une comparaison par histogramme d'un test comportemental chez le rat juvénile, soumis au test de labyrinthe en T, un modèle du TDAH. Sont présentés les résultats en phase « pré-médicament » (colonnes gauches), sous médicament (colonnes centrales), et « post-médicament » (colonnes droites). Le nombre de choix du rat pour la récompense importante mais différée de 30 s est indicateur du niveau d'impulsivité (plus le nombre de choix est importante, moins l'animal est considéré comme impulsif). Le phacétopérane est comparé au méthyl phén idate.

### Description détaillée de l'invention :

### Définitions

Selon l'invention, le terme « traitement » signifie traitement curatif ou prophylactique du TDAH ou d'un de ses symptômes, en particulier le manque d'attention, l'hyperactivité, ou l'impulsivité. Ce terme inclut l'amélioration des symptômes de la maladie.

Le « patient » présentant un TDAH est un humain, enfant, adolescent ou adulte. Plus particulièrement, le patient est un sujet d'intelligence normale, évaluable par un test du Quotient Intellectuel supérieur à 80 (par une évaluation intellectuelle, comme le WISC-IV, par exemple). Le sujet ne présente donc aucune déficience ou retard mental, y compris pas de troubles comportementaux ou moteurs liés à un retard de développement mental (retard intellectuel), comme l'oligophrénie ou l'hyperkinésie d'origine neurologique.

Selon l'invention, un « diastéreoisomère érythro » d'un composé présentant deux carbones asymétriques englobe l'énantiomère (R,S) et l'énantiomère (S,R) dudit composé ainsi que leurs mélanges racémiques.

Un « diastéreoisomère thréo» d'un composé présentant deux carbones asymétriques englobe l'énantiomère (R,R) et l'énantiomère (S,S) dudit composé ainsi que leurs mélanges racémiques.

Dans le cadre de la présente invention, le composé de formule (A) : 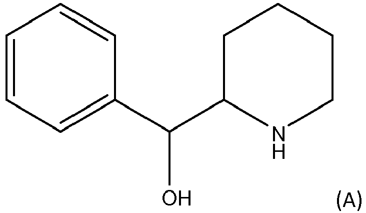 est désigné indifféremment par alpha-phényl-2-pipéridineméthanol ou phényl(pipéridin-2-yl)méthanol, ou encore alpha-phényl(pipéridin-2-yl)méthanol.
On entend par « phacétopérane » le diastéréoisomère thréo de l'acétate de l'alpha-phényl-2-pipéridineméthanol. L'acétate de l'alpha-phényl-2-pipéridineméthanol est également dénommé ci-après acétate de phényl(pipéridyn-2-yl)méthyle ou acétate d'alpha- phényl(pipéridyn-2-yl)méthyle et est représenté par la formule (B).

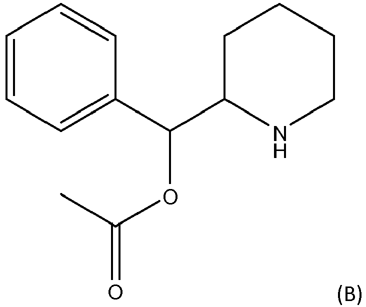

Dans ce qui suit, on entend par lévophacétopérane l'énantiomère lévogyre du phacétopérane c'est-à-dire l'énantiomère (R,R).

De la même manière, on entend par dextrophacétopérane l'énantiomère dextrogyre du phacétopérane c'est-à-dire l'énantiomère (S,S).

### Composés utiles dans l'invention :

Les composés utiles dans l'invention sont l'alpha-phényl(pipéridin-2-yl)méthanol de formule (A), ou ses sels et esters pharmaceutiquement acceptables, seuls ou en mélange.

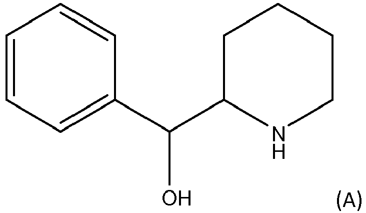

En particulier l'ester sous forme acétate est préféré.

L'acétate de l'alpha-phényl-2-pipéridineméthanol de formule (B) a été synthétisé dans les années 1950 sous le code 7890RP, par les Laboratoires de Recherches de la Société Rhône Poulenc (cf US 2,928,835).

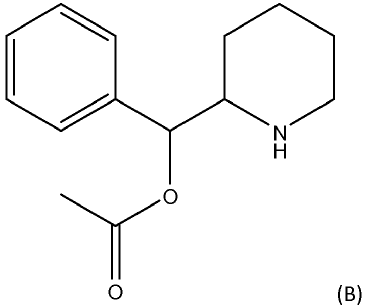

La présente invention concerne les différents diastéréoisomères thréo et érythro de l'alpha-phényl(pipéridin-2-yl)méthanol, leurs sels et leurs esters pharmaceutiquement acceptables qu'ils soient sous forme racémique ou sous forme énantiomérique.

De préférence, la présente invention concerne les diastéréoisomères thréo de ces composés.

Dans un mode de réalisation préféré, la présente invention concerne le diastéréoisomère thréo de l'acétate d'alpha-phényl(pipéridin-2-yl)méthyle, ou l'un de ses sels pharmaceutiquement acceptables, pour une utilisation dans le traitement d'un Trouble Déficit de l'Attention Hyperactivité (TDAH)

Dans un mode particulier de réalisation, on utilise le diastéréoisomère thréo de l'acétate d'alpha-phényl(pipéridin-2-yl)méthyle, ou l'un de ses sels pharmaceutiquement acceptables, sous forme racémique, c'est-à-dire sous la forme d'un mélange de ses énantiomères (S,S) et (R,R).

Dans un autre mode de réalisation, on utilise la forme levogyre du thréo-acétate d'alpha-phényl(pipéridin-2-yl)méthyle ou l'un de ses sels pharmaceutiquement acceptables. La forme lévogyre correspond à l'énantiomère (R,R). Cette forme initialement codée 8228RP, a été ensuite désignée par lévophacétopérane (formule I).

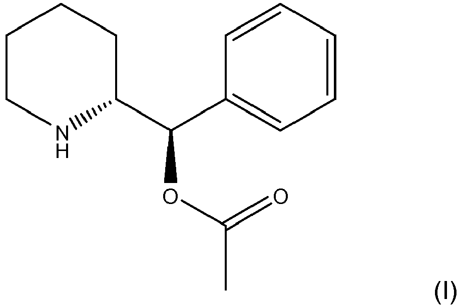

Dans un mode de réalisation supplémentaire, on utilise la forme dextrogyre du thréo-acétate d'alpha-phényl(pipéridin-2-yl)méthyle c'est-à-dire l'énantiomère (S,S), que l'on peut dénommer ici dextrophacétopérane de formule (II).

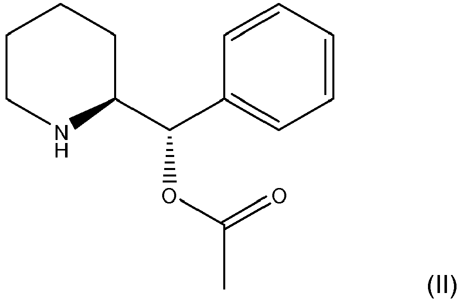

Sans vouloir être lié par une quelconque théorie, le Demandeur est d'avis que l'utilisation de l'énantiomère dextrogyre du phacétopérane ou d'un mélange racémique enrichi en énantiomère dextrogyre du phacétopérane est avantageux lorsque l'on souhaite traiter un TDAH sans provoquer chez le patient un effet sédatif. On entend par « un mélange racémique du phacétopérane enrichi en énantiomère dextrogyre » un mélange pour lequel le pourcentage molaire en énantiomère dextrogyre (c'est-à-dire (S,S)) est supérieur à 50%, de préférence supérieur à 70% et de manière encore plus préférée supérieur à 90%, le pourcentage molaire en énantiomère dextrogyre étant calculé par rapport au nombre de moles totales de phacétopérane dudit mélange.

Un pourcentage molaire en énantiomère dextrogyre supérieur à 90% englobe un pourcentage en énantiomère dextrogyre supérieur à 92%, à 93%, à 94%, à 95%, à 96%, à 97%, à 98%.

De préférence, on utilise dans le cadre de l'invention le dextrophacétopérane, de préférence encore sous la forme d'un sel, par exemple un chlorhydrate.

### Procédé de synthèse des composés de l'invention : ._{'}

Les composés de l'invention peuvent être produits par tout procédé connu de l'homme du métier. L'homme du métier pourra en particulier se référer au brevet US2,928,835 qui décrit l'obtention du phacétopérane sous forme racémique et sous forme lévogyre.

De manière générale, le phacétopérane, ou l'un de ses sels, sous forme racémique ou sous forme d'énantiomère (S,S) ou (R,R), peut être obtenu à partir de l'alpha-phényl-(pipéridin-2-yl)-méthanol sous forme thréo.

Le thréo-alpha-phényl-(pipéridin-2-yl)-méthanol peut être obtenu selon le schéma réactionnel suivant :

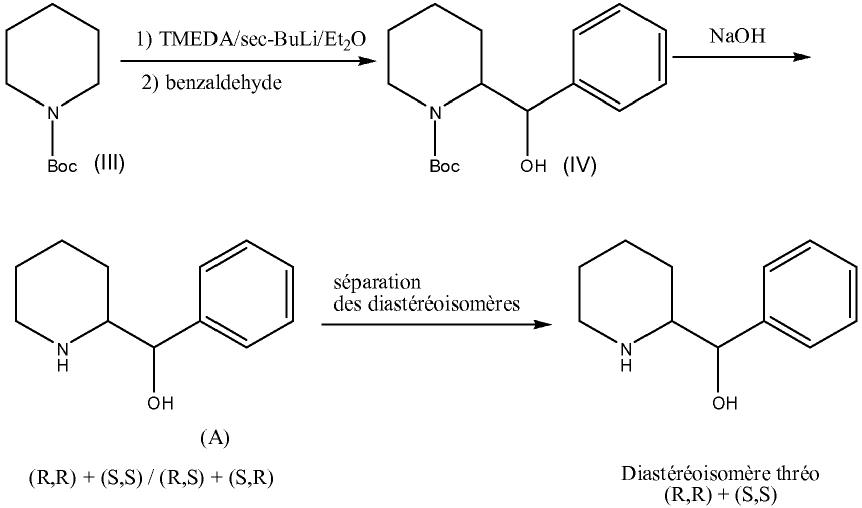

Ce schéma réactionnel comprend les étapes suivantes:
1. l'alpha-lithiation du dérivé N-Boc piperidine (III) suivie d'une substitution électrophile de l'intermédiaire formé en présence du benzaldéhyde de manière à obtenir le dérivé N-(Boc)-α- phenyl(piperidin-2-yl)méthanol (IV). Il va de soi que le groupe Boc peut être remplacé par tout autre groupe protecteur connu de l'homme du métier pour la protection de la fonction amine.
2. Le traitement du dérivé N-(Boc)-α- phenyl(piperidin-2-yl)méthanol (IV) en milieu basique pour obtenir le phenyl(piperidin-2-yl)méthanol sous forme de mélange diastéréoisomérique, et
3. La séparation du diastéréoisomère thréo de l'alpha-phényl-(pipéridin-2-yl)-méthanol du diastéréoisomère érythro qui peut être effectuée par toute méthode séparative adaptée. Il peut s'agir par exemple d'une étape de chromatographie sur colonne de silice du mélange de diastéréoisomères obtenu à l'étape 2 en utilisant un éluant adapté.

Le phacétopérane sous forme racémique peut être obtenu par acétylation du diastéréoisomère de l'alpha-phényl-(pipéridin-2-yl)-méthanol obtenu à l'étape 3.

Le phacétopérane sous forme d'énantiomère (R,R) ou (S,S) peut être obtenu :
- Par dédoublement du phacétopérane sous forme racémique ou
- Par dédoublement du thréo-phényl-(pipéridin-2-yl)-méthanol sous forme racémique suivi par l'acétylation du ou des énantiomères isolés.

Le dédoublement du phacétopérane ou du thréo-phényl-(pipéridin-2-yl)-méthanol peut être réalisé par des méthodes classiques de résolution énantiomérique, par exemple, par chromatographie sur une phase stationnaire chirale ou par cristallisation préférentielle à l'aide d'un agent de dédoublement, généralement un composé chiral tel que l'acide tartrique, l'acide camphorsulfonique, l'acide dibenzoyltartrique ou encore la N-acétyl leucine.

A titre d'exemple, le brevet US2,928,835 décrit la séparation des énantiomères (S,S) et (R,R) du thréo-phényl-(pipéridin-2-yl)-méthanol par cristallisation fractionnée à l'aide de l'acide (-)dibenzoyltartrique dans le propanol. Dans les conditions spécifiques décrites dans US2928835, l'énantiomère dextrogyre reste en solution alors que l'énantiomère lévogyre précipite sous forme de sel de dibenzoyltartrate. De la même manière, il est attendu que l'utilisation de l'acide (+)-dibenzoyltartrique permette la cristallisation sélective de l'énantiomère dextrogyre du phacétopérane. Le Demandeur a montré que la réduction du dérivé Boc de la phényl-(pipéridin-2-yl)-méthanone par le tri-sec-butylborohydrure de lithium (L-Selectride@) permet d'obtenir un intermédiaire clé pour l'obtention du dextrophacétopérane, à savoir, l'alpha-phényl-(pipéridin-2-yl)-méthanol sous forme d'énantiomère (S,S), éventuellement sous forme protégé.

Le présent fascicule décrit également un procédé de préparation du composé sous forme d'énantiomère (S,S) de formule (VI) : 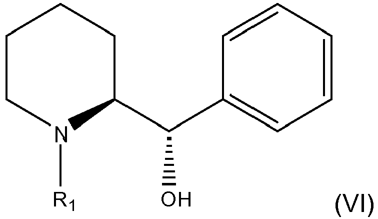 dans laquelle R₁ représente un hydrogène ou un groupe protecteur, ledit procédé comprenant la réduction du composé de formule (V) 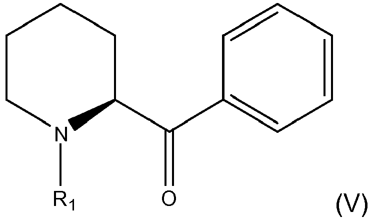 par un sel alcalin de tri-sec-butylborohydrure.

Les sels alcalins de tri-sec-butylborohydrure englobent les sels de lithium, de potassium, et de sodium.

Dans un mode préféré de réalisation, le sel alcalin de tri-sec-butylborohydrure est le tri-sec-butylborohydrure de lithium.

Le groupe protecteur peut être tout groupe protecteur de la fonction amine connu de l'homme du métier. En particulier, il peut s'agir d'un groupe générant un encombrement stérique. Il peut s'agir en particulier du groupement Boc (*tert-*butyloxycarbonyle).

Dans un mode de réalisation particulier de ce procédé, R₁ est un groupe Boc et le sel alcalin de tri-sec-butylborohydrure est le tri-sec-butylborohydrure de lithium.

Le composé de formule (V) correspond à la (S)-phényl-(pipéridin-2-yl)-méthanone sous forme protégée ou non protégée. La (S)-phényl-(pipéridin-2-yl)-méthanone peut être obtenue par toute méthode connue de l'homme du métier.

Le Demandeur a montré que ce composé peut être obtenu de manière avantageuse par réaction d'une amide de Weinreb avec un composé organométallique adapté.

Dans un mode particulier de réalisation, le procédé comprend les étapes suivantes :
(i) La réaction du composé de formule (VII) 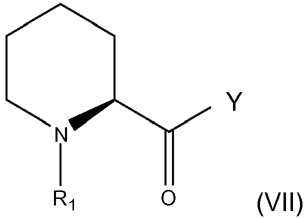
   dans laquelle R₁ représente H ou un groupe protecteur et Y représente OH, F, Cl, Br ou OCH₃,
   avec une N,O-dialkylhydroxylamine de formule NHR₂OR₂ dans laquelle R₂ représente un alkyle, de préférence en C₁-C₆, de manière à obtenir le composé de formule (VIII) 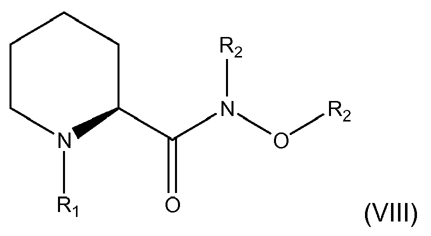
(ii) La réaction du composé de formule (VIII) avec un composé organométallique PhM dans lequel Ph représente un groupe phényle et M représente Li, MgX ou ZnX avec X est un halogénure choisi parmi I, Br et Cl de manière à obtenir le composé de formule (V) 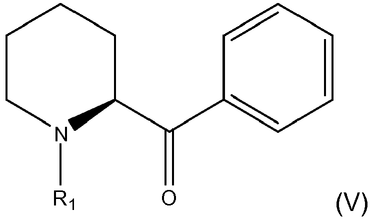
(iii) La réduction du composé de formule (V) par un sel alcalin de tri-sec-butylborohydrure pour obtenir l'alcool de formule (VI) ou un sel de celui-ci, sous forme d'énantiomère (S,S)

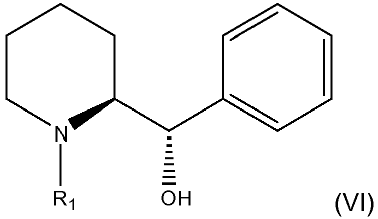

Dans certains modes de réalisation du procédé l'étape (i) du procédé est caractérisée par une, plusieurs ou toutes les caractéristiques suivantes :
- Y est OH,
- R₂ est Me, et
- PhM est le phényl lithium

A l'étape (i), lorsque le composé de formule (VII) est un acide carboxylique (Y = OH), la réaction entre ledit acide carboxylique et la N,O-dialkylhydroxylamine peut être réalisée à l'aide d'un ou plusieurs agents de couplage peptidique tels que les carbodiimides et les sels de phosphonium. On peut citer à titre d'exemple d'agents de couplage peptidique, l'hexafluorophosphate de benzotriazole-1-yl-oxy-tris-(diméthylamino)-phosphonium.

Il va de soi que chaque étape du procédé peut être mise en oeuvre en présence d'un solvant et, éventuellement, en présence d'une base ou d'un acide. Le procédé de préparation du composé de formule (VI) sous forme d'énantiomère (S,S) peut être avantageusement utilisé dans la préparation de l'acétate de l'alpha-phényl(pipéridin-2-yl)méthanol sous forme d'énantiomère (S,S).

Ainsi, un procédé de préparation de l'énantiomère (S,S) de l'acétate de l'alpha-phényl(pipéridin-2-yl)méthanol (dextrophacétopérane) ou de l'un de ses sels pharmaceutiquement acceptables, peut donc comprendre les étapes suivantes
- la préparation du composé de formule (VI) sous forme d'énantiomère (S,S) telle que précédemment exposée, et
- l'acétylation du composé de formule (VI), éventuellement suivie par la déprotection de la fonction amine du groupe pipéridine lorsque R₁ est un groupe protecteur.

En d'autres termes, le procédé de préparation de l'énantiomère (S,S) de l'acétate d' alpha-phényl(pipéridin-2-yl)méthyle (dextrophacétopérane) ou de l'un de ses sels pharmaceutiquement acceptables peut comprendre les étapes suivantes :
- La réduction par un sel alcalin de tri-sec-butylborohydrure du composé de formule (V) 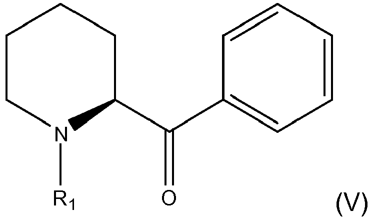 dans laquelle R₁ représente H ou un groupement protecteur, pour obtenir l'alcool ou un sel de celui-ci, sous forme d'énantiomère (S,S) de formule (VI) : 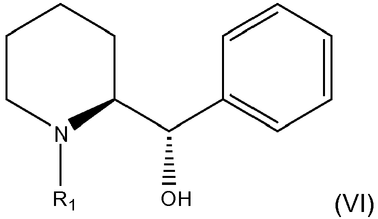
- L'acétylation de l'alcool de formule (VI), éventuellement suivie par la déprotection de la fonction amine du groupe pipéridine lorsque R₁ est un groupe protecteur.

L'étape d'acétylation peut être réalisée par des méthodes bien connues de l'homme du métier, en particulier, en faisant réagir l'alcool de formule (VI) avec l'anhydride acétique ou le chlorure d'éthanoyle.

En ce qui concerne les groupes protecteurs de la fonction amine ainsi que les méthodes permettant leur introduction ou leur clivage, on pourra se référer à l'ouvrage de référence de Greene (Greene's Protective Groups in Organic Synthesis", 2006, John Wiley & Sons Inc; 4ème édition). Si R₁ est un groupement Boc, l'étape de déprotection du groupe pipéridine peut être réalisée par hydrolyse acide par exemple en présence d'acide chlorhydrique ou d'acide trifluoroacétique.

Dans un mode particulier de réalisation, le procédé comprend les étapes suivantes :
(i) La réaction du composé de formule (VII) 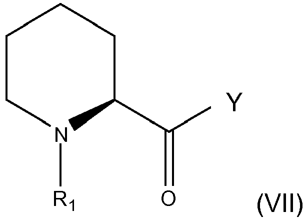
   dans laquelle R₁ représente H ou un groupe protecteur et Y représente OH, F, Cl, Br ou OCH₃,
   avec une N,O-dialkylhydroxylamine de formule NHR₂OR₂ dans laquelle R₂ représente un alkyle, de préférence en C₁-C₆, de manière à obtenir le composé de formule (VIII) 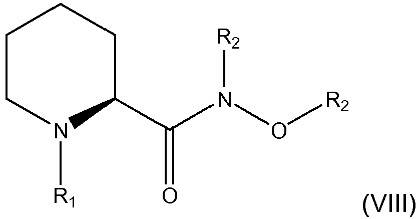
(ii) La réaction du composé de formule (VIII) avec un composé organométallique PhM dans lequel Ph représente un groupe phényle et M représente Li, MgX ou ZnX avec X est un halogénure choisi parmi I, Br et Cl de manière à obtenir le composé de formule (V) 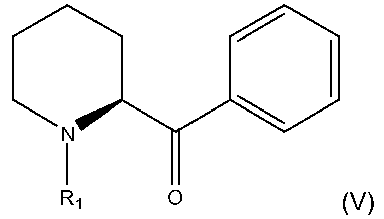
(iii) La réduction du composé de formule (V) par un sel alcalin de tri-sec-butylborohydrure pour obtenir l'alcool de formule (VI) ou un sel de celui-ci, sous forme d'énantiomère (S,S) 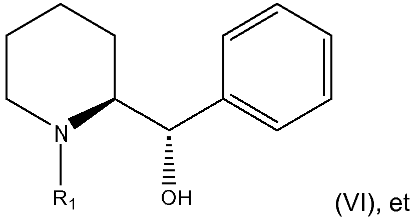
(iv) L'acétylation de l'alcool de formule (VI), éventuellement suivie par la déprotection de la fonction amine du groupe pipéridine quand R₁ représente un groupe protecteur, pour obtenir l'énantiomère (S,S) de l'acétate de l'alpha-phényl(pipéridin-2-yl)méthanol.

Les modes particuliers de réalisation décrits pour la préparation du composé de formule (VI) sous forme d'énantiomère (S,S) sont transposables au procédé de préparation du dextrophacétopérane.

Il va de soi que chaque étape du procédé peut être mise en oeuvre en présence d'un solvant et, éventuellement, en présence d'une base ou d'un acide.

### Formulation

La présente invention fournit une composition pharmaceutique comprenant un composé décrit plus haut, seul ou en mélange, et un véhicule pharmaceutiquement acceptable.

Les compositions selon l'invention peuvent être administrées de différentes manières et sous différentes formes. Ainsi, ils peuvent être administrés de manière systémique, par voie orale, par inhalation ou par injection, comme par exemple par voie intraveineuse, intra-musculaire, sous-cutanée, trans-dermique, intra-artérielle, etc., les voies intraveineuse, intra-musculaire, sous-cutanée, orale et par inhalation étant préférées. Pour les injections, les composés sont généralement conditionnés sous forme de suspensions liquides, qui peuvent être injectées au moyen de seringues ou de perfusions, par exemple. A cet égard, les composés sont généralement dissous dans des solutions salines, physiologiques, isotoniques, tamponnées, etc., compatibles avec un usage pharmaceutique et connues de l'homme du métier. Ainsi, les compositions peuvent contenir un ou plusieurs agents ou véhicules choisis parmi les dispersants, solubilisants, stabilisants, conservateurs, etc. Des agents ou véhicules utilisables dans des formulations liquides et/ou injectables sont notamment la méthylcellulose, l'hydroxyméthylcellulose, la carboxyméthylcellulose, le polysorbate 80, le mannitol, la gélatine, le lactose, des huiles végétales, l'acacia, etc.

Les composés peuvent également être administrés sous forme de gels, huiles, comprimés, suppositoires, poudres, gélules, capsules, aérosols, etc., éventuellement au moyen de formes galéniques ou de dispositifs assurant une libération prolongée et/ou retardée. Pour ce type de formulation, on utilise avantageusement un agent tel que la cellulose, des carbonates ou des amidons.

Dans un mode de réalisation préféré, le composé est utilisé sous une forme appropriée à une administration par voie orale.

De préférence il peut être sous une forme appropriée à une libération prolongée.

De préférence encore, il peut être encore sous forme d'une association ou d'un mélange de microgranules à libération immédiate, et de microgranules à libération prolongée.

### Indications thérapeutiques

L'invention concerne le traitement d'un trouble de l'attention, plus particulièrement du Trouble Déficit de l'Attention Hyperactivité (TDAH), par l'alpha-phényl(pipéridin-2-yl)méthanol, ou ses sels et esters pharmaceutiquement acceptables, en particulier le dérivé acétate, plus particulièrement le dextrophacétopérane.

Le composé peut être administré en monothérapie ou en association avec un ou plusieurs autres principes actifs, y compris des psychostimulants (comme le méthylphénidate ou l'amphétamine).

De manière générale, les composés de l'invention peuvent être utiles pour traiter chez tout sujet (adulte ou enfant), les troubles du sommeil ou du maintien de l'éveil (narcolepsie, hypersomnie), troubles de l'humeur, troubles du comportement (agitation, instabilité), le trouble oppositionnel (avec ou sans provocation), les troubles anxieux, les troubles de la personnalités (trouble envahissant du développement, états limites, schizophrénie), la maladie d'Alzheimer, les démences du sujet âgé (démence fronto-temporale, cortico-basale, maladie des Corps de Lewy), la maladie de Parkinson, le syndrome de Gilles de la Tourette, le tremblement essentiel, et le Syndrome des Jambes Sans Repos (SJSR).

Il est enfin décrit une méthode de traitement de ces troubles ou maladies, en particulier du Trouble Déficit de l'Attention Hyperactivité (TDAH), chez un patient nécessitant un tel traitement, ladite méthode comprenant l'administration audit patient d'une quantité thérapeutiquement efficace d'alpha-phényl(pipéridin-2-yl)méthanol, ou ses sels et esters pharmaceutiquement acceptables, en particulier le dérivé acétate, plus particulièrement le dextrophacétopérane.

Pour le traitement d'un trouble de l'attention, et en particulier du TDAH, des doses journalières d'environ 5 à 1000mg, de préférence environ 5 à 500mg, de préférence encore 5 à 100mg, de préférence encore environ 5 à 30mg, 5 à 20mg, ou 5 à 10mg, sont préférées. De préférence des doses fractionnées sont administrées au patient.

Les exemples et figures illustrent l'invention sans en limiter la portée.

### 1. Exemple de synthèse de chlorhydrate de dextrophacétopérane :

Le chlorhydrate de dextrophacétopérane a été obtenu selon le schéma de synthèse suivant sous la forme d'une poudre blanche avec un rendement total de 20% à 25% et une pureté supérieure à 97%.

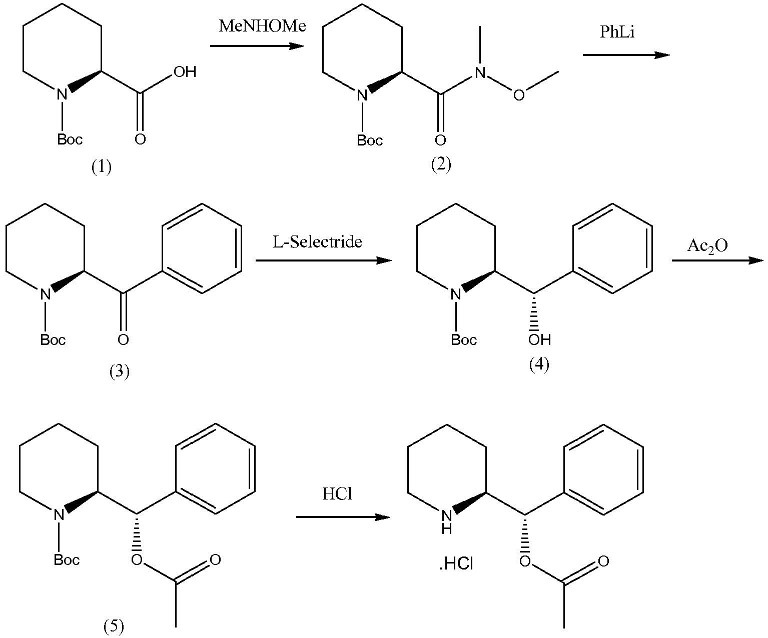

Sous atmosphère d'azote, l'acide (1) (0,5 g, 2,13.10⁻³ mole) est dissous dans CH₂Cl₂. A cette solution, on ajoute le chlorhydrate de *N*-O-diméthylhydroxylamine (0,254g, 2,55.10⁻³ mole) et la triéthylamine (7,51.10⁻³ mole). On ajoute alors l'héxafluorophosphate de benzotriazole-1-yl-oxy-tris-(dimethylamino)-phosphonium (BOP) (1,06g, 2,343.10⁻³ mole) et le milieu réactionnel est agité durant 6h. Le milieu réactionnel est ensuite dilué dans CH₂Cl₂ et transféré dans une ampoule à brome contenant HCl 1 M. La phase organique est lavée à NaHCO₃, puis NaCl saturé, et enfin à l'eau. On sèche sur Na₂SO₄ puis après filtration et évaporation du solvant, l'huile obtenue est purifiée par flash chromatographie (cyclohexane/acétate d'éthyle 8 :2) pour conduire à l'hydroxamate (2) (0,479g, 83%).

L'hydroxamate (2) (0,470g, 1,72.10⁻³ mole) est dissous dans l'éther éthylique, mis sous atmosphère d'azote et refroidi à -23°C. Le phényllithium (1,8M dans dibutyléther, 0,955 mL, 1,72.10⁻³ mole) est ensuite ajouté. Après 3h d'agitation, le milieu réactionnel est ajouté à une solution de KH₂PO₄ 1 M avec de la glace pilée. La phase aqueuse est extraite à l'acétate d'éthyle, séchée, filtrée et évaporée. L'huile obtenue est purifiée par flash chromatographie (cyclohexane/acétate d'éthyle 8 :2) pour conduire à la cétone (3) (0,253g, 52%).

La cétone (3) (0,365g, 1,26.10⁻³ mole) dans du THF anhydre et sous une atmosphère d'azote est refroidie à -78°C. L-Selectride (1 M dans THF, 3,79 mL, 3,78.10⁻³ mole) est alors ajouté. Après 5h d'agitation à cette température, de l'eau puis de l'eau oxygénée à 35% sont ajoutés au milieu réactionnel qui est agité ensuite pendant 2h. La solution obtenue est ensuite diluée dans de l'eau et de l'acétate d'éthyle et la phase aqueuse extraite par de l'acétate d'éthyle. Les phases organiques réunies sont lavées avec de l'eau, puis NaCl saturé, séchées sur Na₂SO₄, puis filtrées et enfin évaporées. L'huile obtenue est purifiée par flash chromatographie (cyclohexane/acétate d'éthyle 8 :2) pour conduire à l'alcool (4) (0,277g, 76%).

L'alcool (4) (0,265g, 0,91.10⁻³ mole), la triéthylamine (0,378 mL, 2,73.10⁻³ mole), la 4-DMAP (5,55 mg, 4,55.10⁻⁵ mole) et l'anhydride acétique (0,516 mL , 5,46.10⁻³ mole) sont mis sous agitation à température ambiante durant 14h. La solution est ensuite basifiée avec 10% K₂CO₃ et extraite avec de l'éther éthylique. Les phases organiques réunies sont séchées sur Na₂SO₄, puis filtrées et évaporées. Le produit obtenu est purifié par flash chromatographie (cyclohexane/acétate d'éthyle 8 :2) pour conduire à l'acétate (5) (0,291g, 95%).

L'acétate (5) (5,7 mg, 1,71.10⁻⁵ mole) est dissous dans l'éther éthylique et l'acide chlohydrique 12N (0,02 mL) est alors ajouté. Après 15 minutes d'agitation la solution est évaporée et séchée à la pompe à palette pour conduire au dextrophacétopérane (4 mg, 87%) sous forme d'une poudre blanche.

Le produit obtenu présente les caractéristiques suivantes:
RMN ¹H (300 MHz, MeOD), δ en ppm : 7,46 (m, 5H), 5,73 (d, J=9Hz, 1 H), 3,69 (m, 1H), 3,50 (d, J=12Hz, 1H), 3,36 (eau dans méthanol deutéré), 3,11 (t, J=9Hz, 1H), 2,18 (s,3H), 1,52-1,97 (m, 6H)
RMN ¹³C (75 MHz, MeOD), δ en ppm: 171,2 ; 136,9 ; 130,5 ; 130,1 (2C) ; 128,6 (2C) ; 77,5 ; 60,5 ; 46,3 ; 26,5 ; 23,2 ; 22,5 ; 20,9.
IR (cm⁻¹): 1741, 1223, 1023, 769, 703
Pouvoir rotatoire : [α]⁵⁸⁹_{D} = +40 (c=0,05 MeOH),

### Excès énantiomérique > 90%

CCM : Rf = 0.43 avec éluant DCM/MeOH
Spectrométrie de masse (ESI) : 234 ([M+1]+

### 2. Exemple de test biologique : Etude comportementale du rat soumis au test de labyrinthe en T, un modèle du TDAH

### 2.1. Modèle :

Le test comportemental labyrinthe en T permet de mesurer la capacité à attendre que manifeste l'animal juvénile (ici, le rat) lorsqu'il est amené à choisir entre une récompense alimentaire rapide et immédiate ou une récompense importante mais différée. Ce test permet d'apprécier la capacité d'attente, c'est-à-dire le niveau d'impulsivité. Chez les rats Wistar jeunes, le méthylphénidate (ritaline®) semble augmenter le nombre de choix pour la récompense importante mais différée. Ce résultat est aussi celui qui est retrouvé avec d'autres médicaments stimulants amphétaminiques (amphétamine), avec les inhibiteurs de la recapture de la noradrénaline « non-stimulants » (atomoxetine, mazindol), mais aussi avec les agents eugrégoriques (modafinil, lauflumide).

Etant donné que ces médicaments réduisent les symptômes (comme l'impulsivité du TDAH), le test du labyrinthe en T, chez les animaux juvéniles est approprié pour tester l'amélioration du contrôle de l'impulsivité par des médicaments pour le traitement du TDAH.

### 2.2. Objectif :

Montrer si le phacétopérane, administré par voie intrapéritonéale (IP) à dose équivalente de celle du méthylphénidate améliore la capacité d'attente chez les jeunes rats Wistar soumis au test du labyrinthe en T. Une amélioration de la capacité d'attente par ce composé indiquerait que ce composé réduit l'impulsivité et par conséquent pourrait être utile dans le traitement du TDAH. Le méthylphénidate est utilisé en tant que produit de référence dans cette expérience.

### 2.3. Protocole :

Les expériences ont été réalisées entre 8h et 18h à température ambiante (22 ± 1,5°C) sous éclairage artificiel dans des conditions calmes.

On a utilisé des rats AF Wistar males (Centre d'Elevage René Janvier, France), de 22 jours au début de l'expérience et entre 30 et 42 jours au moment de l'administration des molécules.

Les molécules testées sont :
- Méthylphénidate (MPH) : 3 mg/kg par voie intrapéritonéale (IP)
- Phacétopérane (composé sous forme racémique désigné BLK-010 dans ce test) : 1 mg/kg par voie intrapéritonéale (IP)
- placebo : 1 mL/kg par voie intrapéritonéale (IP)

Les expériences ont été réalisées dans deux dispositifs de labyrinthe en T identiques en tube de plastique gris opaque (diamètre interne: 7,5 cm), consistant en une allée de départ (30 cm de long), une boîte en plastique transparent (10 cm de large, 10 cm de profondeur, 10 cm de haut) et deux bras (35 cm de long) chacun menant à une boîte rectangulaire en plastique noir (18 cm de large, 30 cm de profondeur, 10 cm de haut). Cf **Figure 1****.** Des portes guillotine amovibles en plastique gris peuvent être insérées dans des fentes verticales, situées à l'entrée de l'allée de départ et à chaque extrémité des bras. Une des boîtes-but (gauche ou droite, suivant les rats) est constamment fournie d'une récompense importante, l'autre d'une petite récompense. Des récompenses importante et petite consistent, respectivement, en 5 et 1 boulettes (20 mg, Technical & Scientific Equipment GmbH, Germany). Les boulettes sont placées dans une coupelle translucide avant chaque essai.

### Phases d'entraînement

*1^{ère} phase- accoutumance.* Les animaux sont d'abord soumis à deux à six sessions de 5 min d'accoutumance. Le rat est délicatement introduit dans l'allée de départ, qui est ensuite fermée avec une porte guillotine insérée dans la fente. On laisse l'animal explorer librement le dispositif et manger des récompenses placées dans des coupelles.
*2^{nde} phase-pré-entraînement.* Après qu'une porte ait été placée dans la fente c2 près de chaque boîte-but, le rat est introduit dans l'allée de départ. Quand il entre dans un des deux bras, une porte est insérée derrière lui dans la fente c1 près de la zone de choix et la porte placée dans la fente c2 est ôtée. Dès que l'animal entre dans la boîte-but, la porte est replacée dans la fente c2. Le rat est récupéré de la boîte-but dès qu'il a mangé les boulettes. L'animal est ensuite remis dans sa cage pour 2 à 3 min. Chaque rat est soumis une à trois fois/jour à cinq sessions d'essais. En 4 à 12 sessions, le rat choisit le bras donnant accès à la récompense importante dans plus de 80% des essais. L'entraînement débute alors.
*3ème phase: entraînement.* Dès lors que les rats suivent 1 à 4 fois/jour des séances d'entraînement de 5 essais pendant lesquels un délai est introduit avant l'accès à la récompense importante. Après qu'une porte ait été placée dans la fente c2 près de chaque boîte-but, le rat est introduit dans l'allée de départ. Quand il entre dans un des deux bras, une seconde porte est insérée derrière lui dans la fente c1 près de la zone de choix, de sorte que le rat choisissant le bras menant à la récompense importante puisse être détenu dans ce bras pendant 30 s -le délai d'attente- avant d'avoir accès au renforcement. Sinon, si l'animal choisit le bras menant à la petite récompense, la porte placée dans la fente c2 est immédiatement ouverte, permettant à l'animal d'entrer dans la boîte-but. Tester les médicaments commence quand l'animal choisit la récompense importante et retardée de 30 s à 2 essais sur 5 (ou moins) pendant deux sessions consécutives et à 1 essai sur 5 (ou moins) à la session suivante. Les animaux qui n'entrent pas dans ce critère pendant 12 sessions sont éliminés de l'expérience.

Les phases de tests s'effectuent de la façon suivante :
2 sessions de contrôle « pré-médicament » (« cont-pre »),
2 sessions de "medicament",
2 sessions de contrôle « post-médicament » (« cont-post »).

Deux sessions de contrôle pré-médicament sont réalisées le même jour, 2 à 4h d'intervalle. Les sessions médicament 1 et 2 sont réalisées un et deux jours, respectivement, après les sessions de contrôle pré-médicament. Les deux sessions de contrôle post-médicament sont réalisées un jour après la session de médicament 2. Le BLK-10, le méthylphénidate ou le placebo sont administrés avant chaque session de médicament.

Les animaux sont répartis au hasard en 3 groupes (n = 6 animaux/groupe) et reçoivent un total de deux administrations IP (une avant chaque session de médicament) de:
- groupe méthylphénidate (3 mg/kg) 30 min avant le test,
- groupe BLK-010 (1 mg/kg) 30 min avant le test

### 2.4 Résultats

L'analyse des données a été réalisée pour chaque animal et le pourcentage de choix de « récompense importante mais différée » calculé de même.

Les résultats sont rapportés dans le Tableau 1.

**Tableau 1.**

| | | **Cont-Pré** | **Médicament** | **Cont-Post** |
|---|---|---|---|---|
| **Placebo IP-60 min** | **Moyenne** | **1,17** | **1,17** | **1,50** |
| | Ecart-type | 0,31 | 0,40 | 0,34 |
| | ≠ *vs*. *Cont-Pre: p=* | | *1,000* | *0,465* |
| (n = 6) | ≠ *vs*. *Cont-Post: p=* | *0,465* | *0,465* | |
| **Methylphenidate 3 mg/kg IP-30 min** | **Moyenne** | **1,17** | **3,67** | **0,83** |
| | Ecart-type | 0,31 | 0,33 | 0,17 |
| | ≠ *vs. Cont-Pre: p=* | | 0,007 | *0,363* |
| (n = 6) | ≠ *vs. Cont-Post: p=* | *0,363* | *0,001* | |
| **Phacétopérane (BLK-010) 1 mg/kg IP-30 min** | **Moyenne** | **1,17** | **4,17** | **0,33** |
| | Ecart-type | 0,31 | 0,65 | 0,21 |
| | ≠ *vs. Cont-Pre: p=* | | ***0,003*** | ***0,042*** |
| (n = 6) | ≠ *vs. Cont-Post: p=* | ***0,042*** | ***0,001*** | |

Le méthylphénidate, utilisé en tant que produit de référence et le BLK-010 sont administrés avant le test. Ces deux molécules améliorent la capacité d'attente chez les jeunes rats Wistar soumis au test du labyrinthe en T par rapport au contrôle.

Le composé BLK-010 apparaît significativement et statistiquement plus aussi efficace que le méthylphénidate dans la capacité d'attente chez les jeunes rats Wistar soumis au test du labyrinthe en T.

Le composé BLK-010 est la seule molécule qui permet d'améliorer significativement le comportement du rat juvénile entre le « pré-traitement » et le « post-traitement » et apparaît significativement et statistiquement plus aussi efficace que le méthylphénidate dans la capacité d'attente chez les jeunes rats Wistar soumis au test du labyrinthe en T (*p*=*0.042*).

L'amélioration de l'impulsivité est retrouvée chez tous les animaux (n=6) traités par le composé BLK-010 (phacétopérane) (1 mg/kg). Celle-ci est significativement meilleure à celle retrouvé avec le méthylphénidate (3 mg/kg) (cf **Figure 2**).

Le phacétopérane (1 mg/kg) n'entraine aucune accoutumance, ni même de dépense (avant et après traitement). Il est robuste dans le temps (au cours des différents essais) et se montre toujours plus efficace que le méthylphénidate (3 mg/kg).

## Revendications

1. Alpha-phényl(pipéridin-2-yl)méthanol, ou ses sels et esters pharmaceutiquement acceptables, pour une utilisation dans le traitement d'un Trouble Déficit de l'Attention Hyperactivité (TDAH).

2. Acétate d'alpha-phényl(pipéridin-2-yl)méthyle sous forme thréo (phacétopérane) ou un de ses sels pharmaceutiquement acceptables, pour une utilisation selon la revendication 1, dans le traitement d'un TDAH.

3. Acétate d'alpha-phényl(pipéridin-2-yl)méthyle sous forme thréo (phacétopérane) ou un de ses sels pharmaceutiquement acceptables, pour une utilisation selon la revendication 2, dans le traitement d'un TDAH, ledit phacétopérane étant sous sa forme dextrogyre.

4. Acétate d'alpha-phényl(pipéridin-2-yl)méthyle sous forme thréo (phacétopérane) ou un de ses sels pharmaceutiquement acceptables, pour une utilisation selon la revendication 2, dans le traitement d'un TDAH, ledit phacétopérane étant sous sa forme levogyre.

5. Alpha-phényl(pipéridin-2-yl)méthanol, ou ses sels et esters pharmaceutiquement acceptables, en particulier le dérivé acétate, ou un de ses sels pharmaceutiquement acceptables, pour une utilisation selon l'une des revendications 1 à 4, dans le traitement d'un TDAH chez l'adulte.

6. Alpha-phényl(pipéridin-2-yl)méthanol, ou ses sels et esters pharmaceutiquement acceptables, en particulier le dérivé acétate, ou un de ses sels pharmaceutiquement acceptables, pour une utilisation selon l'une des revendications 1 à 5, sous une forme appropriée à une administration par voie orale.

7. Alpha-phényl(pipéridin-2-yl)méthanol, ou ses sels et esters pharmaceutiquement acceptables, en particulier le dérivé acétate, ou un de ses sels pharmaceutiquement acceptables, pour une utilisation selon la revendication 6, sous une forme appropriée à une libération prolongée.

8. Alpha-phényl(pipéridin-2-yl)méthanol, ou ses sels et esters pharmaceutiquement acceptables, en particulier le dérivé acétate, ou un de ses sels pharmaceutiquement acceptables, pour une utilisation selon la revendication 6, sous forme d'une association ou d'un mélange de microgranules à libération immédiate, et de microgranules à libération prolongée.

## Patentansprüche

1. Alpha-Phenyl(Piperidin-2-yl)methanol oder dessen pharmazeutisch unbedenkliche Salze und Ester für eine Verwendung bei der Behandlung einer Aufmerksamkeitsdefizit-/Hyperaktivitätsstörung (ADHS).

2. Alpha-Phenyl(Piperidin-2-yl)methylacetat in Threo-Form (Phacetoperan) oder eines von dessen pharmazeutisch unbedenklichen Salzen für eine Verwendung nach Anspruch 1 bei der Behandlung einer ADHS.

3. Alpha-Phenyl(Piperidin-2-yl)methylacetat in Threo-Form (Phacetoperan) oder eines von dessen pharmazeutisch unbedenklichen Salzen für eine Verwendung nach Anspruch 2 bei der Behandlung einer ADHS, wobei das Phacetoperan in seiner rechtsdrehenden Form vorliegt.

4. Alpha-Phenyl(Piperidin-2-yl)methylacetat in Threo-Form (Phacetoperan) oder eines von dessen pharmazeutisch unbedenklichen Salzen für eine Verwendung nach Anspruch 2 bei der Behandlung einer ADHS, wobei das Phacetoperan in seiner linksdrehenden Form vorliegt.

5. Alpha-Phenyl(Piperidin-2-yl)methanol oder dessen pharmazeutisch unbedenkliche Salze und Ester, insbesondere das Acetatderivat, oder eines von dessen pharmazeutisch unbedenklichen Salzen, für eine Verwendung nach einem der Ansprüche 1 bis 4 bei der Behandlung einer ADHS bei Erwachsenen.

6. Alpha-Phenyl(Piperidin-2-yl)methanol oder dessen pharmazeutisch unbedenkliche Salze und Ester, insbesondere das Acetatderivat, oder eines von dessen pharmazeutisch unbedenklichen Salzen, für eine Verwendung nach einem der Ansprüche 1 bis 5 in einer für eine orale Verabreichung geeigneten Form.

7. Alpha-Phenyl(Piperidin-2-yl)methanol oder dessen pharmazeutisch unbedenkliche Salze und Ester, insbesondere das Acetatderivat, oder eines von dessen pharmazeutisch unbedenklichen Salzen, für eine Verwendung nach Anspruch 6 in einer für eine länger anhaltende Freisetzung geeigneten Form.

8. Alpha-Phenyl(Piperidin-2-yl)methanol oder dessen pharmazeutisch unbedenkliche Salze und Ester, insbesondere das Acetatderivat, oder eines von dessen pharmazeutisch unbedenklichen Salzen, für eine Verwendung nach Anspruch 6 in Form einer Verbindung oder einer Mischung aus Mikrokörnchen mit sofortiger Freisetzung und aus Mikrokörnchen mit länger anhaltender Freisetzung.

## Claims

1. Alpha-phenyl(piperidin-2-yl)methanol, or the pharmaceutically acceptable salts and esters thereof, for use in the treatment of an attention deficit hyperactivity disorder (ADHD).

2. Alpha-phenyl(piperidin-2-yl)methyl acetate in the threo form (phacetoperane) or a pharmaceutically acceptable salt thereof, for use according to Claim 1, in the treatment of ADHD.

3. Alpha-phenyl(piperidin-2-yl)methyl acetate in the threo form (phacetoperane) or a pharmaceutically acceptable salt thereof, for use according to Claim 2, in the treatment of ADHD, said phacetoperane being in its dextrorotatory form.

4. Alpha-phenyl(piperidin-2-yl)methyl acetate in the threo form (phacetoperane) or a pharmaceutically acceptable salt thereof, for use according to Claim 2, in the treatment of ADHD, said phacetoperane being in its laevorotatory form.

5. Alpha-phenyl(piperidin-2-yl)methanol, or the pharmaceutically acceptable salts and esters thereof, in particular the acetate derivative, or a pharmaceutically acceptable salt thereof, for use according to one of Claims 1 to 4, in the treatment of ADHD in adults.

6. Alpha-phenyl(piperidin-2-yl)methanol, or the pharmaceutically acceptable salts and esters thereof, in particular the acetate derivative, or a pharmaceutically acceptable salt thereof, for use according to one of Claims 1 to 5, in a form suitable for administration by the oral route.

7. Alpha-phenyl(piperidin-2-yl)methanol, or the pharmaceutically acceptable salts and esters thereof, in particular the acetate derivative, or a pharmaceutically acceptable salt thereof, for use according to Claim 6, in a form suitable for prolonged release.

8. Alpha-phenyl(piperidin-2-yl)methanol, or the pharmaceutically acceptable salts and esters thereof, in particular the acetate derivative, or a pharmaceutically acceptable salt thereof, for use according to Claim 6, in the form of a combination or of a mixture of microgranules for immediate release, and of microgranules for prolonged release.
